# EUROPEAN PATENT APPLICATION

(11) **EP 4 252 637 A1**
(43) Date of publication of application: **04.10.2023**
(21) Application number: 23166384.0
(22) Date of filing: 03.04.2023
(51) Int. Cl.: A61B 5/03, A61B 5/00, A61B 8/00

(54) **METHOD AND APPARATUS FOR NON-INVASIVE DETERMINING INTRAORBITAL AND INTRACRANIAL COMPLIANCE VALUES**

(30) Priority: 01.04.2022 US 202263326352 P
(71) Applicant: Kaunas University of Technology, 44249 Kaunas (LT)
(72) Inventor: RAGAUSKAS, Arminas, 51362 Kaunas (LT); PETKUS, Vytautas, 48300 Kaunas (LT); CHALECKAS, Edvinas, 50374 Kaunas (LT); ZAKELIS, Rolandas, 47131 Kaunas (LT); BARTUSIS, Laimonas, 53363 Kaunas district (LT); PUTNYNAITE, Vilma, 50382 Kaunas (LT); HAMARAT, Yasin, 53363 Kaunas district (LT); KRAKAUSKAITE, Solventa, 51422 Kaunas (LT); DEIMANTAVICIUS, Mantas, 49212 Kaunas (LT); TAMOSUITIS, Tomas, 45137 Kaunas (LT)
(74) Representative: Sayettat, Julien Christian

(57) **Abstract**

The invention is directed generally to a method and apparatus for non-invasively determining and intraorbital and intracranial compliance values in mammals including humans.

## Description

### FIELD OF INVENTION

The present invention is directed generally to a method and apparatus for non- invasively determining intraorbital and intracranial compliance values measurement.

### BACKGROUND OF THE INVENTION

Intracranial compliance (ICC) has been studied with investigations of intracranial pressure (ICP) in neurocritical care to help predict brain function deterioration. These ICC studies presently measure ICC using invasive methods.

ICC is a parameter of ICP that is measured using an intracranial pressure to intracranial volume (ΔV) relationship. The measurement units for ICC are in ml/mmHg units and is equal to a ratio of ΔV/ΔICP. Intraorbital compliance (IOC) is defined similarly to ICC but it is applicable to pressure and volume in the patient's eye orbit.

In order to invasively measure intracranial or intraorbital compliance values an invasive bolus injection into intracranial or intraorbital media is needed in order to create a micro-change ΔV in intracranial or intraorbital volume and also invasive monitoring over a time period of ICP(t) or of intraorbital pressure (IOP) over a time period, IOP(t), is needed in order to identify Δ ICP or Δ IOP as the reactions to change Δ V. The invasive pressure - volume relationship based method is also used for IOC measurements.

In the present state of the art, an invasive Spiegelberg Compliance Monitor type apparatus is used which involves the use of an intraventricular small pneumatic balloon that induces continuous repetitive volumetric impulses into the brain. The apparatus simultaneously records the invasive ICP(t) changes and periodically calculates ICC values as Δ V change divided by ΔICP change.

Under the existing methods in the art the only non-invasive method to directly or indirectly identify ICC values is use of a phase-contrast MRI based pressure-volume apparatus and accounting. One key disadvantage of MRI based methods is impossibility to perform accurate and precise measurements when traumatic brain injury patients are being treated in intensive care units.

The existing prior art methods do not disclose a non-invasive ultrasonic intracranial compliance value measurement method or apparatus.

### BRIEF SUMMARY OF THE INVENTION

Specific features of the invention are as follows, the invention includes a novel method and apparatus for non-invasive intracranial compliance ICC value measurement by applying a hermetically sealed external pressure Pe applicator with close to zero internal compliance to the eye through closed eye lid of the human and by identifying a balance between the measured IOC(Pe) and ICC(ICP) values.

First, there is a connection of a patient's skull with a hermetically sealed external pressure applicator integrated with ultrasonic transducer of transorbital Doppler device or ultrasonic triplex scanner. Then the pressure applicator is filled with degasified water in order to achieve close to zero internal compliance, is applied to the patient's eye. When the pressure applicator is applied to the closed eye lid of the patient, the eye lid is isolated from the internal media (water) of the pressure applicator by a thin elastic non-allergenic and ultrasonically transparent film. The pressure applicator is connected with a water micro-volume pump and micro - volume Δ V meter/monitor. Inside the pressure applicator there is a connection of the internal medium of the pressure applicator with the pressure Pe sensor and a connection with the pressure Pe meter.

Similarly, the apparatus and pressure applicator have component connections to a micro-volume pump, ΔV meter, pressure Pe meter and TOD device (or triplex scanner) with a microprocessor based control unit to control the components of the apparatus. The control unit controls the micro-volume pump, monitors ΔV(t) and ΔPe(t), calculates IOC(t)=ΔV(t)/ΔPe(t) and records blood flow pulsatility indexes PI and RI in both selected segments of the OA. The control unit also compares both pulsatility indexes in real time and stops Pe(t) increment and IOC(t) decrement at the moment ti when IOC(ti)=ICC. The control unit displays the measured ICC value on ICC meter's display.

The invention is an apparatus for non-invasively determining an intracranial compliance value measurement for a mammal, the apparatus includes a pressure applicator integrated with an ultrasonic transducer of a transorbital Doppler device or an ultrasonic triplex scanner. The pressure applicator is filled with an internal media with approximately zero internal compliance. The apparatus included a thin film configured to separate the internal media of the pressure applicator from the eyelid of the mammal when the pressure applicator is applied to the mammal's eyelid. The invention also includes a media pump and a volume meter connected to the pressure applicator, and a pressure sensor and pressure meter connected to the internal media of the pressure applicator. The apparatus includes a TOD device connected to the ultrasonic transducer. The control unit is connected to the media pump, the volume meter, the pressure meter and the TOD device or the ultrasonic triplex scanner. The control unit of the apparatus is configured to control the media pump, the volume meter and pressure meter and to incrementally change the pressure measured by the pressure sensor by incrementally changing the volume of the media in the pressure applicator. The control unit is configured to monitor changes in media volume over time and changes in the media pressure over time and configured to calculate IOC(t)=ΔV(t)/ΔPe(t) and to record blood flow pulsatility indexes PI and RI in both selected intracranial and intraorbital segments of the OA. The control unit is also configured to compare both pulsatility indexes in real time when applied to the mammal's eyelid and stop the incremental changes in pressure applied to a mammals eye at the moment ti when IOCi(ti)=ICC. The control unit is also configured to display the measured ICC value on a display.

### BRIEF DESCRIPTON OF THE FIGURES

Figures 1a and 1b are drawings of one embodiment of the apparatus for non-invasive intracranial compliance measurement according to the invention.
Figure 2 is a flow chart and algorithm of one embodiment of the non-invasive intracranial compliance measurement method according to the invention.
Figures 3a and 3b are representations of one embodiment of pressure applicator according to the invention.
Figure 4 is an illustration of one embodiment of the pressure applicator on a closed eye lid of a human according to the invention.
Figures 5a and 5b are graphs of *in vivo* animal test data that identifies relationships of IOP(Pe) and IOC(Pe).
Figures 6a and 6b are graphs of *in vivo* tests results on a human showing relationship of the area of the optic near and subarachnoid space around the optic nerve depending on external pressure applied to eye lid of the human.
Figure 7 is an illustration of one embodiment of the pressure applicator according to the invention.

### DETAILED DESCRIPTION OF THE INVENTION

The invention includes a method and apparatus for non-invasively determining an intracranial compliance value measurement in a mammal. The mammals include, among other mammals, piglets and humans. The following abbreviations are used in the application:
ICC - intracranial compliance value in
   ml/mmHg,
ICP - intracranial pressure in mmHg,
IOC - intraorbital compliance value in
   ml/mmHg,
IOP - intraorbital pressure in mmHg
OA - ophthalmic artery.
Pe - external pressure applied to the closed eye lid by pressure applicator,
PI = (Vs-Vd)/(Vs+Vd) - pulsatility index of OA blood flow in OA segment of interest, Vs - systolic and Vd - diastolic blood flow velocities,
RI = (Vs - Vd)/Vs - resistivity index of OA flow in OA segment of interest.
TBI - traumatic brain injury.
TOD - transorbital Doppler two depth blood flow velocity measurement device,
V - intracranial or intraorbital volume in ml,

The inventors, using *in vivo* experiments with application of external pressure Pe(t) to a human's eye's orbit show that it is possible to transfer Pe into IOP with a high correlation between IOP and Pe and good linearity of IOP(Pe) relationship.

It is also possible to control and to change IOC values in a wide range by changing Pe. The invention includes a novel method and apparatus for *non-invasive* intracranial compliance ICC value measurement by applying a hermetically sealed external pressure Pe applicator with close to zero internal compliance to the eye through closed eye lid of the human and by identifying a balance between the measured IOC(Pe) and ICC(ICP) values.

According to one embodiment of the invention, to achieve a balanced ICC being equal IOCi(Pei) indication the invention contemplates using simultaneous two depth measurement by ultrasonic transorbital Doppler device (TOD) of blood flow pulsatility in intracranial and extracranial (intraorbital) segments of an ophthalmic artery (OA). Pulsatilities are the same in both intraorbital and extraorbital OA segments when ICC = IOCi(Pei) where Pei is a discrete Pe value at the time moment ti when balance ICC = IOC is achieved. In order to identify such balance for ICC value measurement purposes a pressure applicator's internal compliance has to be close to zero and Pe has to be increased step by step pressure increases from Pe = 0 up to Pe = Pei. For example, during testing, Pe steps of 1 mmHg was used during *in vivo* experiments. The close to zero internal compliance of the pressure Pe applicator was achieved by using non-compressible liquid (degasified water) as an internal medium of pressure Pe applicator.

Under the inventive method, the pressure applicator 10 is filled with non-compressible degasified water 30 and is applied to the closed eyelid 50 of a mammal, such as a human patient. The eye lid 50 is isolated from water 30 by a thin elastic non-allergenic film 60. The pressure applicator 10 is hermetically sealed to the human patient's face around the eye 55 to form two rigid boxes (applicator and cranium) which are firmly and hermetically sealed and joined together when the pressure applicator is applied to the patient's face around the patient's eye. Embodiments of the pressure applicator 10 could use different types of hermetic seals (62) including ones using a rigid plastic ring as part of the hermetic seal.

When applied to the patient, the rigid box pressure applicator has an internal compliance equal to IOC because the compliance of water is close to zero and the other rigid box, formed by the patient's skull or cranium has internal compliance equal to ICC. The ophthalmic artery crosses the internal media of both "boxes" and pulsatility of blood flow in intracranial and intraorbital segments of the OA depend on ICC and IOC values. The IOC is controlled by the volume of water and Pe(t) changes inside the pressure applicator.

IOC is identified by ratio ΔV of water and ΔPe. Increasing V(t) and Pe(t) using step by step incremental changes causes similar step by step incremental decreases to IOC. The TOD device monitors blood flow velocity pulsations in both segments of OA simultaneously during the process of IOC(V(t)) change over time.

The IOC change process stops when balance is achieved when IOC is approximately equal to ICC and the ICC value has been identified and presented on the ICC meter's display and control unit 100.

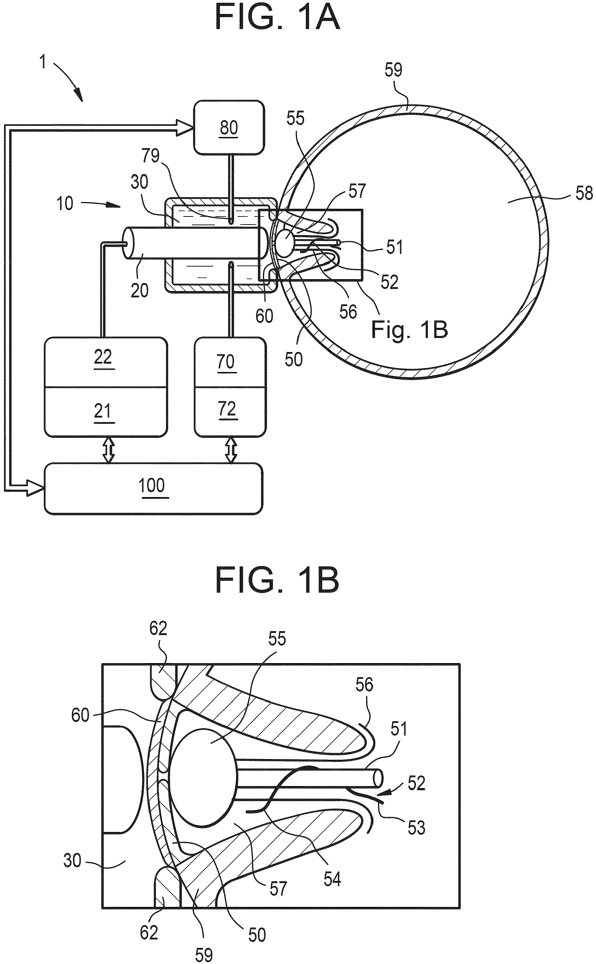

Figure 1 shows one embodiment of the structure of the apparatus 1 for non- invasive intracranial compliance measurement. Fig 1(A) shows the components of one embodiment of the inventive apparatus in relation to a human's eye 55. FIG. 1(B) shows an exploded view of the inventive apparatus when applied to the human's closed eyelid 50. FIG. 1(A) illustrates the connection of a human skull 50 with a hermetically sealed external pressure applicator integrated with an ultrasonic transducer 20 having a transorbital Doppler device 21 and ultrasonic triplex scanner 22. The transorbital Doppler device, TOD 21, is a transorbital Doppler two depth blood flow velocity measurement device. The inventive apparatus can include a pressure applicator 10 filled with degasified water 30 in order to achieve close to zero internal compliance. One difference between the invention and the prior art is in the structure and operation of the pressure applicator applied to a patient's eye and the close to zero compliance of pressure applicator's internal media - non-compressible degasified water.

**The** invention also includes isolation of the closed eye lid 50 of a human patient from the internal media 30 of the pressure applicator by a thin elastic non-allergenic and ultrasonically transparent film 60. Pressure applicator 10 is connected to a water micro-volume pump 70 and micro - volume ΔV monitor 72 which controls and maintains the volume and pressure of the media in the pressure applicator.

The internal medium 30 of the pressure applicator 10 is also connected to the external pressure Pe sensor 79 and external pressure Pe meter 80. As external pressure is applied to the closed eyelid 50 of the patient by the pressure applicator 10 the Pe meter 80 measures the external pressure.

The pressure applicator also has connections of the micro-volume pump 70 , the ΔV meter 72, the external pressure Pe meter 80 and the TOD device 21 (or triplex scanner) with the microprocessor based control unit 100. The control unit 100 controls the micro-volume pump 70 and monitors the measure ΔV(t) from the ΔV meter 72 and ΔPe(t) measured by the Pe meter 80. The control unit 100 then calculates IOC(t)=ΔV(t)/ΔPe(t) and records blood flow pulsatility indexes PI and RI in both the intracranial section segment 53 of the ophthalmic artery 51 and the intraorbital segment 54 of the ophthalmic artery 51 show in FIG 1(B).

The control unit 100 compares both pulsatility indexes in real time and stops Pe(t) increment and IOC(t) decrement at the moment ti when IOCi(ti)=ICC. The control unit 10 displays measured ICC value on ICC meters display.

FIG. 1(B) shows the exploded view of the pressure applicator 10 being applied to the human's closed eyelid 50. FIG. 1(B) includes the general relationship between the ultrasonic transducer 20, the internal media 30, the thin film 60 of the pressure device 10 which can be a thin elastic non-allergenic film and the human's closed eyelid 50 and eyeball 55. Also shown in the figure is the human's skull 59, the dura 56, the eye orbit 57, the optic nerve 51 and the intracranial segment 53 and intraorbital segment 54 of the ophthalmic artery. The pressure applicator 10 includes a mechanical seal 62 that is applied to the human's eye and skull for hermetically sealing the pressure applicator 10 to the human's face.

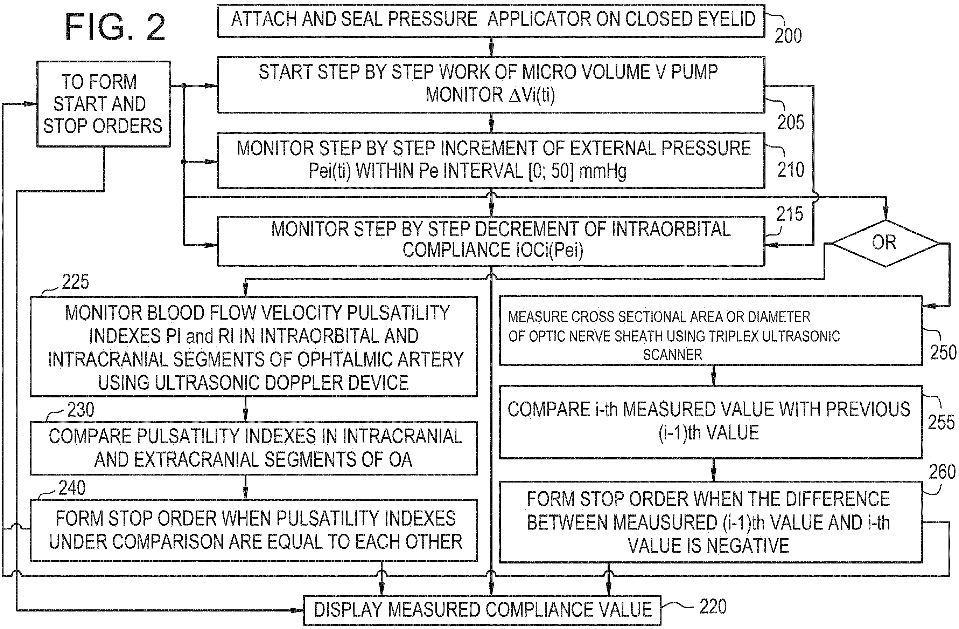

Figure 2. shows an algorithm of the inventive method for a non-invasive intracranial compliance measurement process. The inventive method starts with attaching and sealing a pressure applicator 10 to a human's closed eyelid 50. Then a step by step, intermittent process occurs by periodically operating the micro Volume V pump 205, to measure and monitor ΔVi(ti) the change in volume over time. Monitoring of the step by step incremental increases of external pressure Pei(ti) typically begins with a Pe interval 0 to 50 mmHg 210. Measurements are performed by increasing the Pe starting at 0 mmg and increasing the pressure in incremental steps of between 1 and 5 mmHg per step. The total pressure exerted can exceed 50 mmHg as a result of the incremental increases. This is followed by the monitoring of the step by step decrement of intraorbital compliance IOCi(Pei) 215. During this monitoring the measured compliance value is displayed 220 on the control unit display 100.

While the above steps take place the invention also contemplates monitoring the blood flow velocity pulsatility indexes PI and RI in intraorbital 54 and intracranial 53 segments of ophthalmic artery 52 using ultrasonic doppler device 20, 225. Then there is a comparison of the pulsatility indexes in intracranial 53 and extracranial segments 54 of the OA 230. A stop order to stop the step by step pressure increases is then issued when the pulsatility indexes under comparison are equal to each other 240. The displayed measured compliance values can then be displayed by a display 220.

Alternatively, or conjunction with the other measurements contemplated by the invention, there is a measurement of the cross-sectional area or diameter of the optic nerve sheath using a triplex ultrasonic scanner 250. Next, that measurement is compared with the optic nerve sheath diameter with its normal diameter value 255. The measurement is repeatedly taken with increased Pe and each measured value is compared with the previous measured value (comparing the i-th measured value with previous (i-1)th measured value. A stop order is formed when the difference of measured optic nerve sheath diameter is equal to the optic nerve sheath's normal diameter value 260. The stop order is identified when the difference between the measured (i-1)the value and i-th value is negative. A displayed measured compliance value can then be displayed by the display 220.

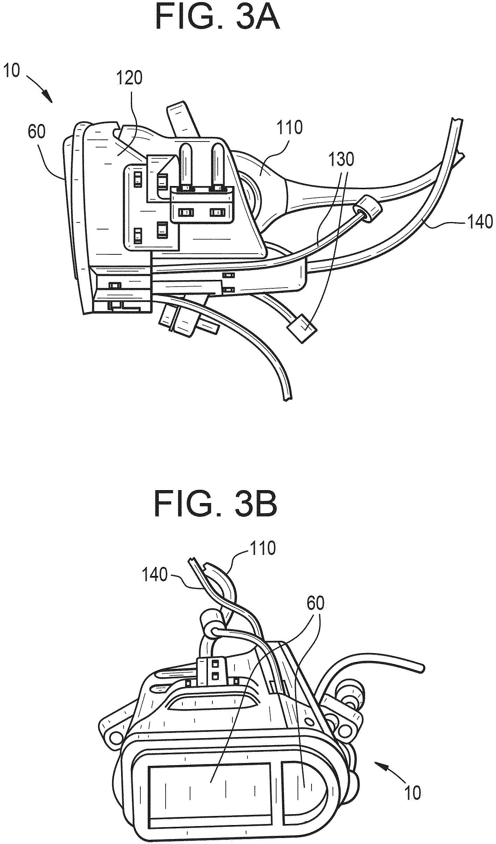

Figure 3. is a pressure applicator 10 with an internal ultrasonic transducer of ultrasonic triplex scanner or transorbital Doppler device. FIG. 3(A) shows a side view of the pressure applicator 10 which contains an internal ultrasonic transducer 20 of ultrasonic triplex scanner or transorbital Doppler device. The embodiment shown in FIG. 3(A) shows the connection for a Matrix 3D ultrasonic transducer 110, and the location for a XL14-3 of triplex ultrasonic scanner EPIQ Elite (Philips) installed into the specially designed pressure applicator 10, 120. The tubes 130 for connection with water micro-volume pump 70 and ΔV meter 72, and the cable 140 for connection of pressure Pe sensor 70 to Pe meter 140 are also shown. FIG 3(b) shows a perspective view of the pressure applicator with the thin elastic non-allergic film 60.

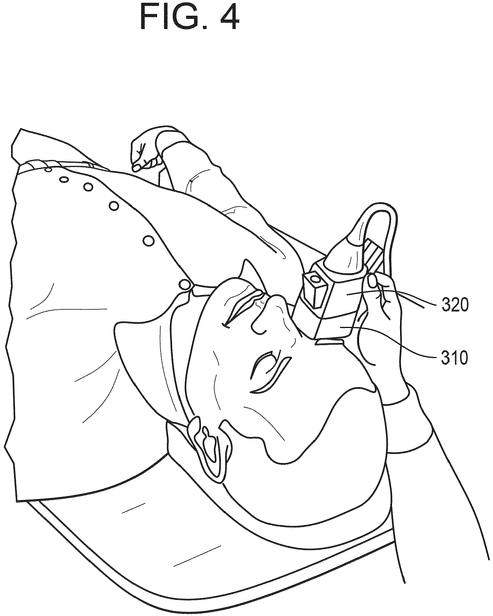

Figure 4. is a representation of one embodiment of the pressure applicator on a closed eye lid of a human. The pressure applicator is shown applied to the eye and skull of a human. The pressure applicator 320 in the embodiment shown is made of rigid plastic and includes an internal mechanical seal 62, 310 for hermetically sealing of the pressure applicator 320 to the human's face. The internal mechanical seal is configured to form a hermetic seal when the pressure applicator is applied with pressure to the human's eye and skull. The internal mechanical seal in this embodiment is covered by the external housing of the pressure applicator and configured with small separate components, or fingers, that form the mechanical seal when pressed up against the human's eye and skull.

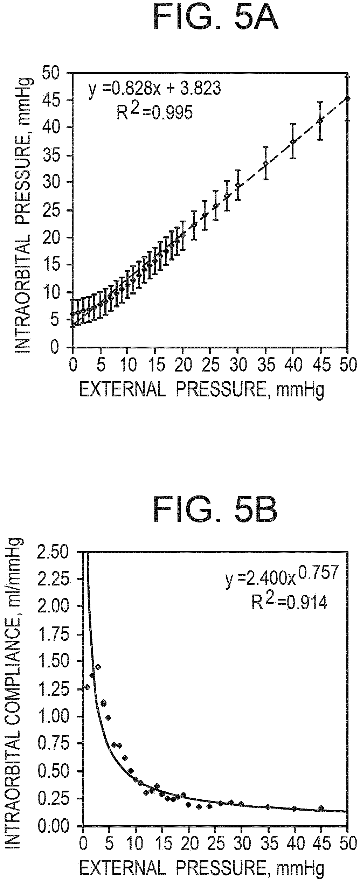

Figure 5. Shows graphs data from *in vivo* animal model testing in piglets using the inventive method and apparatus. The graphs show the relationships of IOP(Pe) and IOC(Pe) measurements during use of the invention. FIG. 5(A) shows IOP(Pe) results of animal testing and FIG 5(B) shows IOC(Pe) results of animal testing. There y(x) is an approximation equation of the relationship IOC(Pe) and R is a correlation factor between IOC and Pe. In FIG 5A the approximation equation of the relationship IOP(Pe) is y=0.828x + 3.823 and the correlation factor R²= 0.995. In FIG 5B the approximation equation of the relationship IOC(Pe) is y=0.2400 x 0.757 and the correlation factor R²= 0.914. The graphs demonstrate that externally applied pressure to the eye (Pe) is linearly transferred to the intraorbital pressure (IOP) as shown in FIG. 5A. Also, the graphs demonstrate that intraorbital compliance (IOC) can be controlled by externally applied pressure (Pe) (FIG. 5B). This is evidence that the balance between intraorbital compliance IOC and intracranial compliance ICC and also the balance between IOP and Pe can be achieved. The algorithm of the invention is based on these findings.

The invention is based on sensitivity of blood flow pulsatilities of intracranial OA segments and intraorbital OA segments to ICC and IOC values. In an alternative embodiment of the invention, a way to identify a balance ICC = IOC is a measurement of the diameter of the optic nerve sheath using a triplex ultrasonic scanner. A measurement of the optic nerve sheath under pressure Pe is taken then that measurement is compared with the optic nerve sheath diameter with its normal diameter value. A stop Pe increment order is generated when the difference of measured optic nerve sheath diameter is equal to the optic nerve sheath's normal diameter value.

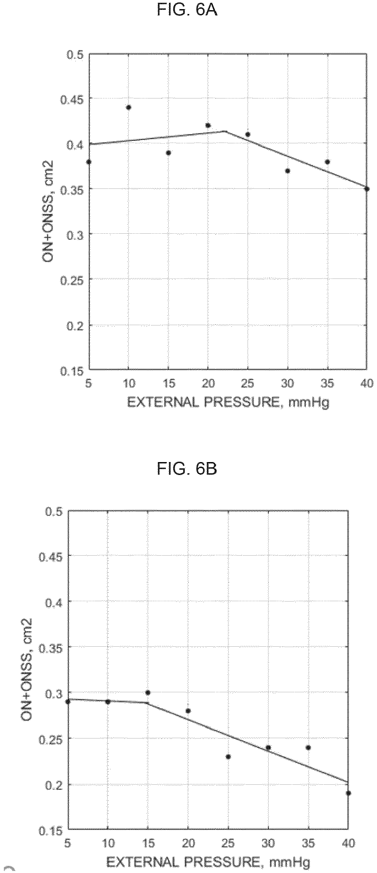

FIG. 6 includes test data from *in vivo* testing on a healthy volunteer human using the inventive method. The graphs show measurement of the sum area of optic nerve (ON) and subarachnoid space around the optic nerve (ONSS) depending on the external pressure applied to the volunteer's eye lid. FIG. 6A show data when a volunteer lying flat on their back is in a bed and has the lower end of the bed at the volunteer's feet raised until the flat bad surface forms a 30° angle with a plane parallel to the original level plane of the bed . It can be seen from the graph that the volunteer's ICP is increased up to 22 mmHg when the volunteer's feet have been raised above their head. FIG. 6B shows data from when the volunteer is lying flat in the supine position and the patient's body is on a level plane at 0°. In this example, the data shows the volunteer's ICP is in the normal range of 12-14 mmHg. Measurements are performed by increasing Pe from 0 mmHg to a certain pressure value until the breakpoint (singularity point) is detected in ON+ONSS graph. (See Figures 6A and 6B). The singularity points are identified when there is a balance between IOP and ICP and also between the intracanial and intraorbital compliances. Fig. 6 shows examples when Pe was incrementally increased with 5 mmHg steps until 40 mmHg was reached until the breakpoint or singularity point was identified. In general, there are no limitations to the upper limit for Pe or to the step size for Pe incrementation, e.g. Pe might be changed from 0 to 50 mmHg (or more). Additionally, the increase in Pe could be in incremental steps ranging from 1 mmHg to 5 mmHg.

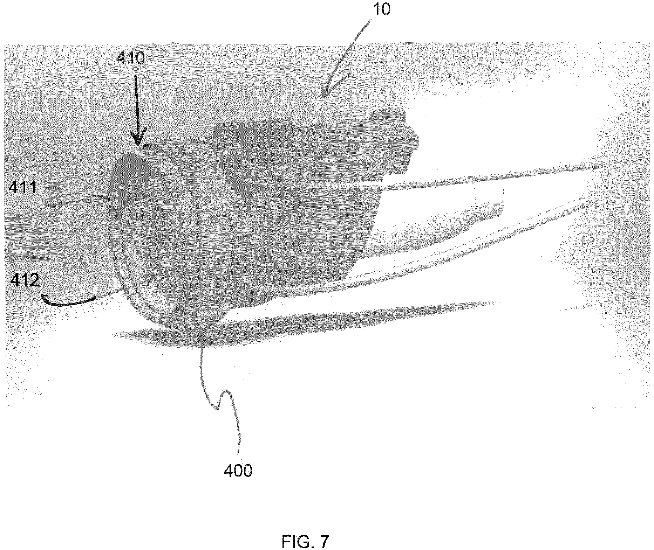

Figure 7. is an another embodiment of the pressure applicator 10 according to the invention with a different type of hermetic seal. Any type of mechanical seal that will form a hermetic seal between the pressure applicator and the human's eye and skull will work in the invention. In this embodiment the pressure applicator 10 includes an external mechanical seal 400. The mechanical seal 400 includes a plurality of fingers 410 forming a ring. The mechanical seal is configured to form a hermetic seal with the human's face when the pressure applicator is applied with pressure to the human's eye and skull. The mechanical seal 400 is easily adaptable to the human face around the eyeball. When the pressure applicator 10 is applied to the human's face the outward end 411 of the fingers 410 contact the human's face. The inward end 412 of the fingers extend toward the body of the pressure applicator 10 and are in contact with a soft sponge-type material that permits each finger to move and adjust which in turn adjusts the shape of the finger ring to the face of the human and to form the mechanical hermetic seal.

In one embodiment an 1.6-2.0 MHz ultrasonic transorbital Doppler device is applied for simultaneous measurements of blood flow velocity pulsatilities in two specific depths of the OA- one at the intracranial segment depth and the other at the intraorbital segment depths.

Although specific advantages of the invention have been enumerated above, various embodiments may include some, none, or all of the enumerated advantages. Other technical advantages may become readily apparent to one of ordinary skill after review of the figures and description. Although exemplary embodiments are illustrated in the figures and described below, the principles of the present disclosure may be implemented using any number of techniques, whether currently known or not. The present disclosure should in no way be limited to the exemplary implementations and techniques illustrated in the drawings and described herein.

## Claims

1. An apparatus for non-invasively determining an intracranial compliance value measurement for a mammal, the apparatus comprising:
A pressure applicator integrated with an ultrasonic transducer of a transorbital Doppler device or an ultrasonic triplex scanner;
the pressure applicator being filled with an internal media with approximately zero internal compliance;
a thin film configured to separate the internal media of the pressure applicator from the eyelid of the mammal when the pressure applicator is applied to the mammal's eyelid;
a media pump and a volume meter connected to the pressure applicator; a pressure sensor and pressure meter connected to the internal media of the pressure applicator;
a TOD device connected to the ultrasonic transducer;
a control unit connected to the media pump, the volume meter, the pressure meter and the TOD device or the ultrasonic triplex scanner;
the control unit being configured to control the media pump, the volume meter and pressure meter and to incrementally change the pressure measured by the pressure sensor by incrementally changing the volume of the media in the pressure applicator;
the control unit being configured to monitor changes in media volume over time and changes in the media pressure over time and configured to calculate IOC(t)=ΔV(t)/ΔPe(t) and to record blood flow pulsatility indexes PI and RI in both selected intracranial and intraorbital segments of the OA;
the control unit is also configured to compare both pulsatility indexes in real time when applied to the mammal's eyelid and stop the incremental changes in pressure applied to a mammals eye at the moment ti when IOCi(ti)=ICC; and
the control unit configured to display the measured ICC value on a display.

2. The apparatus of claim 1 wherein the mammal is a human.

3. The apparatus of claim 1 wherein the external pressure applicator includes a seal configured to hermetically seal the pressure applicator to the mammal's face when applied to the mammals face.

4. The apparatus of claim 1 wherein the internal media is water.

5. The apparatus of claim 1 wherein the internal media is degasified water.

6. The apparatus of claim 1 wherein the thin film is an elastic, non-allergenic and ultrasonically transparent film.

7. The apparatus of claim 1 wherein the media pump is a micro-volume pump and the volume meter is a micro volume ΔV meter and is configured to meter and monitor changes in the media volume.

8. The apparatus of claim wherein the ultrasonic transducer is a triplex ultrasonic scanner configured to measure the measure the cross-section area of the mammal's optic nerve sheath.

9. The apparatus of claim 3 wherein the seal is a mechanical seal and extends externally beyond the end of the pressure applicator.

10. A method for non-invasively determining an intracranial compliance
value measurement of a human, the method comprising; connecting a hermetically sealed pressure applicator integrated with an ultrasonic transducer of transorbital Doppler device or an ultrasonic triplex scanner to the orbit of the skull of human ;
applying pressure to the pressure applicator filled with degasified water in order to achieve close to zero internal compliance;
isolating the closed eye lid of the human from the internal media (water) of pressure applicator by thin elastic non-allergenic and ultrasonically transparent film;
connecting the pressure applicator with a water micro-volume pump and micro - volume deltaV meter/monitor;
connecting the internal medium of pressure applicator with pressure Pe sensor and pressure Pe meter;
connecting the micro-volume pump, ΔV meter, pressure Pe meter and TOD device (or triplex scanner) with microprocessor based control unit;
controlling the micro-volume pump, monitors ΔV(t) and ΔPe(t), calculating IOC(t)=ΔV(t)/ΔPe(t) and recording blood flow pulsatility indexes PI and RI in both selected segments of OA with the control unit;
the control unit comparing both pulsatility indexes in real time and stops Pe(t) increment and IOC(t) decrement at the moment ti when IOCi(ti)=ICC; and
displaying the measured ICC value on ICC meters display.

11. The method of claim 9 wherein the ultrasonic transducer is of an ultrasonic triplex scanner and further including conducting a first measurement of the cross-sectional area of the optic nerve sheath;
applying additional pressure to the pressure applicator;
conducting a second measurement of the cross-sectional area of the optic nerve sheath comparing the second measurement of the cross- sectional area of the optic nerve sheath with the first measurement of the cross-sectional area of the optic nerve sheath;
forming a stop order when the difference between the second measurement of the cross-sectional area of the optic nerve sheath is less than the first measurement of the cross-sectional area of the optic nerve sheath.
